# EUROPEAN PATENT APPLICATION

(11) **EP 4 461 402 A1**
(43) Date of publication of application: **13.11.2024**
(21) Application number: 24167389.6
(22) Date of filing: 28.03.2024
(51) Int. Cl.: B01J 20/04, A61L 9/014, B01D 39/00, B01J 20/06, B01J 20/20, B01J 20/22, B01J 20/28, B01J 20/32, A62B 23/02

(54) **SORBENT BED FOR AN AIR FILTER**

(30) Priority: 30.03.2023 US 202363493273 P; 26.03.2024 US 202418616707
(71) Applicant: Avon Protection Systems, Inc., Cadillac, MI 49601 (US)
(72) Inventor: OJIRI, Michelle, Michigan, 49601 (US); BURRESS, Jacob, Cadillac, 49601 (US)
(74) Representative: Mewburn Ellis LLP

(57) **Abstract**

A sorbent filter (5, 105, 205, 305) and air filter assembly (1) includes at least a first adsorbent layer (10, 110, 210, 310) and a second adsorbent layer (20, 120, 220, 320). The first adsorbent layer (10, 110, 210, 310) can include one of a metal organic framework (MOF) or activated carbon. The second adsorbent layer (20, 120, 220, 320) is positioned adjacent the first adsorbent layer (10, 110, 210, 310) and can include an adsorbent material with water.

## Description

### GOVERNMENT INTEREST

This invention was made with government support under Contract No. W911SR20D0001 awarded by the U.S. Department of Defense. The government has certain rights in the invention.

### TECHNICAL FIELD

The disclosure generally relates to filter assemblies, and particularly to sorbent beds for air filtering.

### BACKGROUND

Fluid filters and devices can be utilized for removing contaminants from fluid streams, including particulates, noxiants, toxic substances, or the like. Such filters can include one or more filter media with a material structure configured to trap contaminants while allowing the fluid stream to pass therethrough. Such filter media can include metals, polymers, composite materials, activated carbon, or the like.

### BRIEF DESCRIPTION

In one aspect, the disclosure relates to a sorbent filter. The sorbent filter includes a first adsorbent layer including at least one of a metal organic framework (MOF) or activated carbon, and a second adsorbent layer positioned adjacent the first adsorbent layer and including an adsorbent material with water in an amount by weight greater than 0%, and less than or equal to 10%.

In another aspect, the disclosure relates to a sorbent filter. The sorbent filter includes a first adsorbent layer including a metal organic framework with water in an amount greater than 0%, and less than or equal to 20%; and a second adsorbent layer positioned adjacent the first adsorbent layer and including activated carbon with water in an amount by weight greater than or equal to 0%, and less than or equal to 10%.

### BRIEF DESCRIPTION OF THE DRAWINGS

A full and enabling disclosure of the present description, including the best mode thereof, directed to one of ordinary skill in the art, is set forth in the specification, which makes reference to the appended figures, in which:
FIG. 1 is a schematic perspective view of an air filter assembly with a sorbent filter in accordance with various aspects described herein.
FIG. 2 is a schematic cross-sectional view of the sorbent filter of FIG. 1 in accordance with various aspects described herein.
FIG. 3 is a plot illustrating filter performance of the sorbent filter of FIG. 1.
FIG. 4 is a plot illustrating filter performance of the sorbent filter of FIG. 1 after applying an aging process to the sorbent filter.
FIG. 5 is a schematic cross-sectional view of another sorbent filter in accordance with various aspects described herein.
FIG. 6 is a schematic cross-sectional view of another sorbent filter in accordance with various aspects described herein.
FIG. 7 is a schematic cross-sectional view of another sorbent filter in accordance with various aspects described herein.

### DETAILED DESCRIPTION

Filter assemblies can be configured to purify a contaminated fluid stream passing through the material by way of trapping contaminants within a filter material. Trapping performance can be affected by a number of factors, including a density of the filter material, a composition of the filter material, an amount of filter material, or an average particle size of the filter material. Generally, the pressure drop (or breathing resistance) through a filter increases or decreases as the density, composition, amount, and/or particle size of filter material changes. In respiratory applications such as full facepiece respirators, filter media are used for removing noxiants or toxic substances from a gas or air stream. Pressure drop through the filter media can become important for maintaining breathability during operation.

Filter assemblies such as sorbent beds can include one or more layers of filter media. Activated carbon is widely used as a filter material. Activated carbon layers can have large airflow resistance at thicknesses needed for certain performance targets, which can be undesirable for respiratory filter applications.

Another material that can be used in a filter assembly or sorbent bed is a metal-organic framework (also referred to herein as "MOF"), which is generally porous and has a crystalline structure. MOFs are also sometimes referred to synonymously as "MOF substances," "MOF materials," or "porous coordination polymers." Such metal-organic frameworks have relatively simple modular structures and form a specific class of porous materials. MOFs generally comprise a mononuclear complex as a crosslinking point or "node" to which a plurality of polyfunctional or polydentate ligands are bound.

Aspects of the disclosure provide for a filter assembly, such as a sorbent bed, which has an improved filtering performance over a wide range of contaminant types, and effective over a longer time duration, compared to typical filters of comparable size. The disclosed filter assembly is suitable for use in purification of gases and/or gas mixtures, including for respiratory filters. For example, the filter assembly disclosed herein can be used for removal of nitrogen dioxide, ammonia, hydrogen cyanide, cyanogen chloride, cyanogen, dimethyl methyl phosphonate, phosgene, sarin, sulfur dioxide, or cyclohexane, in some non-limiting examples..

As may be used herein, the terms "first," "second," "third," "fourth," or the like can be used interchangeably to distinguish one component from another and are not intended to signify location or importance of the individual components. In addition, as may be used herein, a "set" of elements can include any number of such elements, including only one element.

Here and throughout the specification and claims, range limitations are combined, and interchanged. Such ranges are identified and include all the sub-ranges contained therein unless context or language indicates otherwise. All ranges disclosed herein are inclusive of the endpoints, and the endpoints are independently combinable with each other, unless context or language indicates otherwise.

All directional references (e.g., radial, axial, upper, lower, upward, downward, left, right, lateral, front, back, top, bottom, above, below, vertical, horizontal, clockwise, counterclockwise) are only used for identification purposes to aid the reader's understanding of the disclosure, and do not create limitations, particularly as to the position, orientation, or use thereof. Connection references (e.g., attached, coupled, connected, and joined) are to be construed broadly and can include intermediate members between a collection of elements and relative movement between elements unless otherwise indicated. As such, connection references do not necessarily infer that two elements are directly connected and in fixed relation to each other.

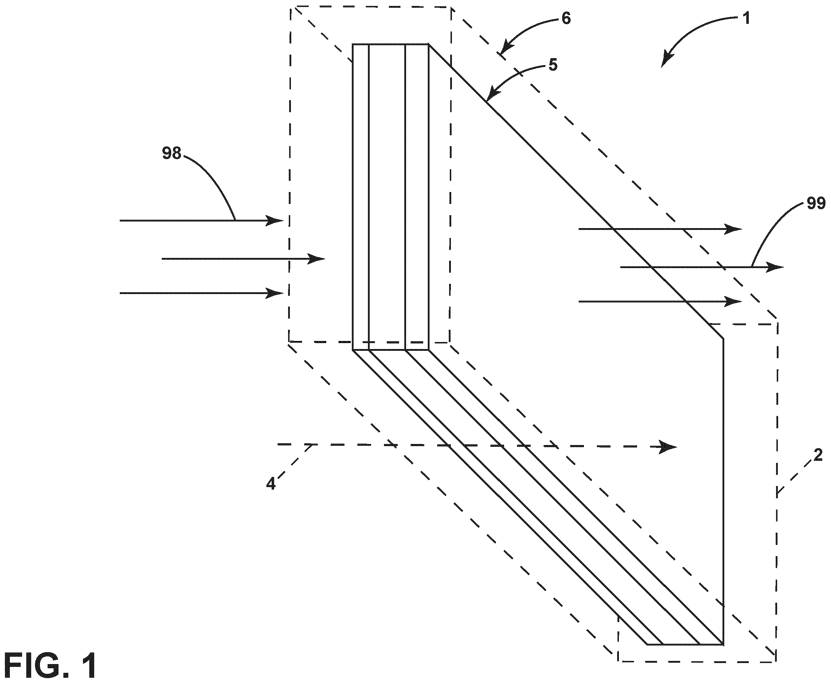

FIG. 1 illustrates an exemplary air filter assembly 1. The air filter assembly 1 includes a housing 2 defining an air flow path 4. A sorbent filter 5 can be positioned within the housing 2. The sorbent filter 5 can include a plurality of filtering layers for adsorption of contaminants from air flowing through the air filter assembly 1. Contaminated air 98 and filtered air 99 are shown entering and exiting, respectively, the air filter assembly 1 along the air flow path 4.

The housing 2 and the sorbent filter 5 are schematically illustrated with a rectangular geometric profile, and it will be understood that the housing 2 and the sorbent filter 5 can have any suitable form. For instance, in one exemplary implementation the housing 2 can at least partially define a filtering facepiece respirator 6, such as a gas mask or a particulate-filtering mask. In another exemplary implementation, the housing 2 can at least partially define a cartridge for use with an air filtering device, such as a removable filter cartridge for a room air filter, a facepiece respirator, or the like. The sorbent filter 5 can be removable from the housing 2 in some examples, or be fixedly secured to the housing 2 in some examples. For instance, the sorbent filter 5 can be in the form of a sorbent bed carried by the housing 2 and insertable into an air filtering device. It is also contemplated that the sorbent filter 5 can be used as a standalone filter in some implementations, or in conjunction with a mechanical particulate filter in some implementations.

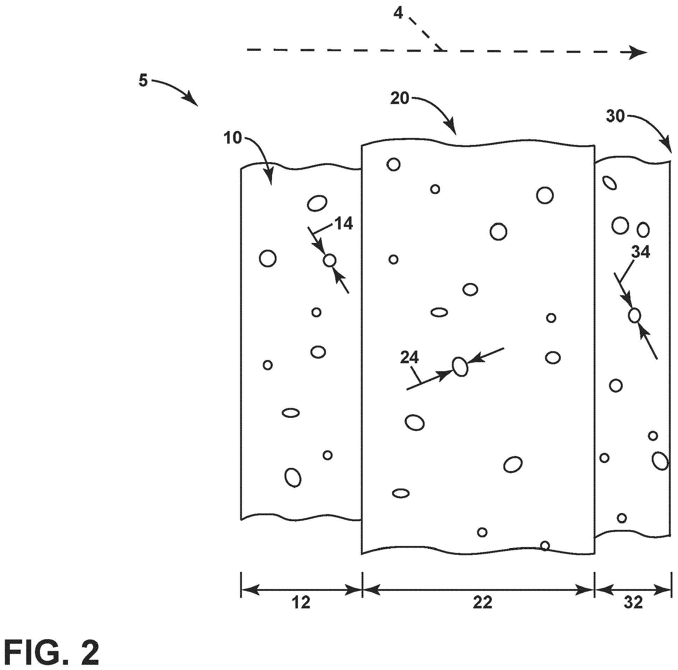

Turning to FIG. 2, a cross-sectional view is shown of the sorbent filter 5 in isolation. The sorbent filter 5 is illustrated having a first adsorbent layer 10, a second adsorbent layer 20, and a third adsorbent layer 30 (also referred to herein as "first layer 10," "second layer 20," and "third layer 30," respectively). While three layers are illustrated in FIG. 2, any number of layers can be provided. As shown, the air flow path 4 extends in a direction from the first layer 10 toward the second layer 20. However, the disclosure is not so limited and the sorbent filter 5 can be arranged with any suitable orientation with respect to the air flow path 4. For instance, it is contemplated that the air flow path 4 can extend through the sorbent filter 5 in a direction from the second layer 20 toward the first layer 10.

The first layer 10, second layer 20, and third layer 30 can define a respective first layer thickness 12, second layer thickness 22, and third layer thickness 32. As shown, the second layer thickness 22 is greater than the third layer thickness 32, which is greater than the first layer thickness 12. It will be understood that the layer thicknesses 12, 22, 32 can have any suitable size, including any suitable relative size. In some non-limiting examples: the first layer thickness 12 can be between 2-6 mm, including 3 mm; the second layer thickness 22 can be between 14-21 mm, including 19 mm; and the third layer thickness 32 can be between 2-10 mm, including 4.5 mm.

In addition, the first layer 10, second layer 20, and third layer 30 are schematically illustrated with particles having a variety of particle sizes. An exemplary first particle size 14, second particle size 24, and third particle size 34 are shown for the respective first layer 10, second layer 20, and third layer 30. In a non-limiting example, any or all of the first particle size 14, second particle size 24, or third particle size 34 can be in a range between 0.212-2.36 mm.

The first layer 10 can include a metal organic framework (MOF). More specifically, it is contemplated that the first layer 10 can include a zirconium-based MOF impregnated with zinc chloride in an amount by weight greater than 0%, and less than or equal to 10%. The zirconium-based MOF can be additionally impregnated with water, in an amount by weight greater than 0%, and less than or equal to 20%. The zirconium-based MOF can be additionally impregnated with a polymeric binder, in an amount by weight greater than 0%, and less than or equal to 10%.

The second layer 20 is positioned adjacent the first layer 10 and includes an adsorbent material. In one non-limiting example, the adsorbent material can include Chemsorb Multigard 3830 from Molecular Products, Inc. More specifically, the adsorbent material can include activated carbon, in an amount by weight greater than or equal to 60%, and less than 100%. The adsorbent material can also include copper II oxide, in an amount by weight greater than 0%, and less than or equal to 10%. The adsorbent material can also include molybdenum, in an amount by weight greater than 0%, and less than or equal to 5%. The adsorbent material can also include triethylenediamine, in an amount by weight greater than or equal to 0%, and less than or equal to 15%. The adsorbent material can further include water, in an amount by weight greater than or equal to 5%, and less than or equal to 10%.

The third layer 30 is positioned adjacent the second layer 20 and is formed of an adsorbent material. More specifically, the third layer 30 can include activated carbon, in an amount by weight greater than or equal to 60%, and less than 100%. The third layer 30 can also include copper carbonate hydroxide, in an amount by weight greater than 0%, and less than or equal to 10%. The third layer 30 can also include zinc oxide, in an amount by weight greater than 0%, and less than or equal to 10%. The third layer 30 can also optionally include water, in an amount by weight greater than or equal to 0%, and less than or equal to 10%. The third layer 30 can further include copper oxide, in an amount by weight greater than 0%, and less than or equal to 10%.

During operation, the contaminated air 98 sequentially passes through the first layer 10, second layer 20, and third layer 30 of the sorbent filter 5. Each of the layers 10, 20, 30 remove contaminants from the contaminated air 98, including gaseous contaminants or particulate contaminants, and the filtered air 99 exits the sorbent filter 5.

In certain contexts where the sorbent filter 5 is utilized with a respiratory device, it is beneficial for the sorbent filter 5 to maintain a minimum air flow rate or breathability during operation while also meeting performance targets for contaminant removal. The added water or moisture in the sorbent filter 5 was found to inhibit dust accumulation during operation, which improves breathability and extends the working lifetime of the sorbent filter 5.

Table 1 below illustrates filter performance results for the sorbent filter 5 with respect to selected agents. In particular, Table 1 provides a summary of performance measures for an exemplary sample of 100 sorbent filters 5 having a first layer thickness 12 of 4.50 mm, a second layer thickness 22 of 19 mm, and third layer thickness 32 of 2.5 mm. The performance measures include: airflow resistance at flow rates of 32 L/min and 85 L/min; penetration, expressed as a percentage of contaminant concentration in the filtered air 99 compared to the contaminated air 98 at a flow rate of 85 L/min; and an overall weight of the sorbent filter 5. It can be appreciated that the sorbent filter 5 maintains breathability at high flow rates while exhibiting excellent adsorption characteristics.

**Table 1**

| | **Resistance @ 32 L/min (mmH₂O)** | **Resistance @ 85 L/min (mmH₂O)** | **Penetration @ 85 L/min (%)** | **Filter Weight (g)** |
|---|---|---|---|---|
| **Minimum** | 14.6 | 40.9 | 0.0001 | 288.2 |
| **Maximum** | 16.3 | 44.9 | 0.0009 | 290.5 |
| **Average** | 15.5 | 43.0 | 0.0008 | 289.5 |
| **Range** | 1.7 | 4.0 | 0.0008 | 2.3 |

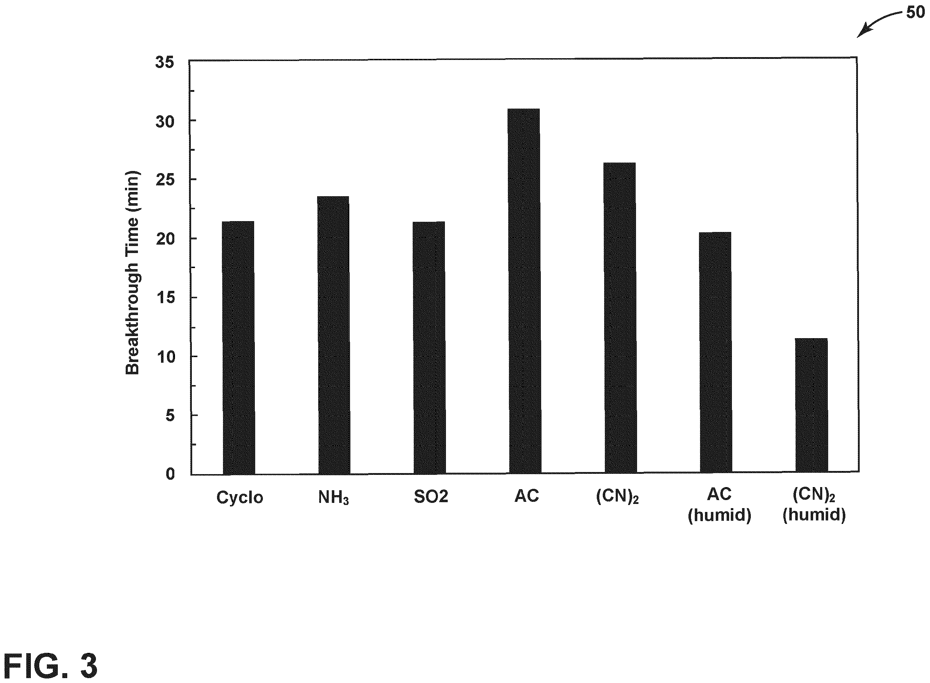

Turning to FIG. 3, a plot 50 illustrates filter performance for the sorbent filter 5 with respect to filtering of selected agents. In particular, breakthrough times for cyclohexane ("cyclo") were tested at a relative humidity of 80%. Breakthrough times for ammonia (NH₃) and sulfur dioxide (SO₂) were tested at a relative humidity of 25%. Breakthrough times for hydrogen cyanide (AC) and cyanogen (CN)₂ were tested at a relative humidity of 50%. Hydrogen cyanide and cyanogen were additionally tested at after being pre-conditioned in a humid environment (denoted "humid" in FIG. 3). For all agents except the pre-conditioned cyanogen test, the sorbent filter 5 demonstrated breakthrough times of 20 minutes or more.

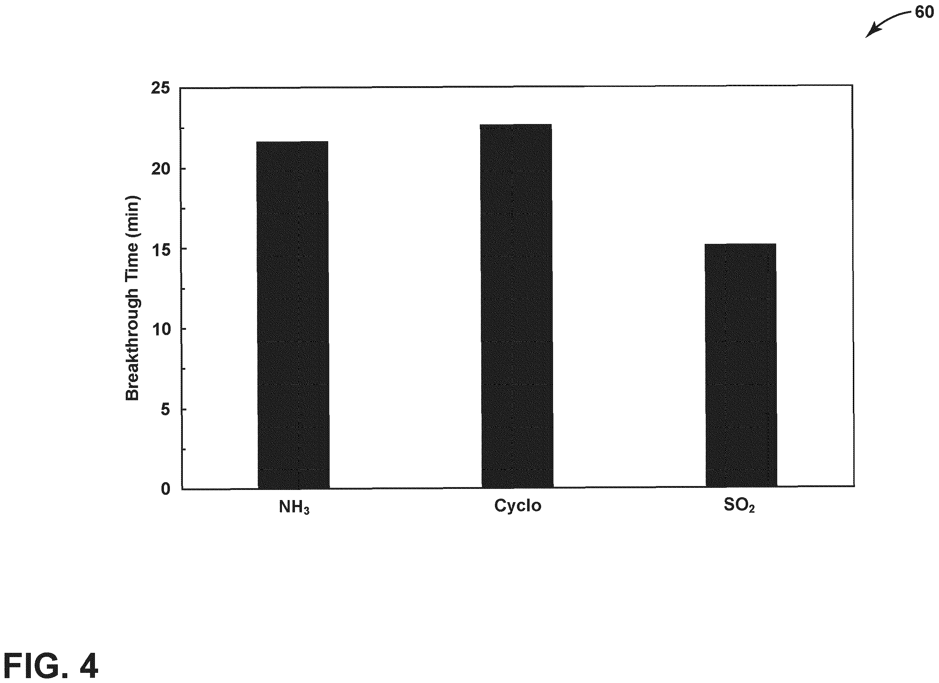

Referring now to FIG. 4, another plot 60 illustrates filter performance for the sorbent filter 5 after an accelerated aging process. In particular, breakthrough times for ammonia, cyclohexane, and sulfur dioxide were tested after applying a 5-year shelf-life accelerated aging process per the Arrhenius equation, which is commonly used to calculate accelerated aging testing conditions and durations. The above-described process includes aging the sorbent filter 5 for 57 days (8.1 weeks) at 71°C to simulate a 5-year shelf life. The aged sorbent filter 5 demonstrated breakthrough times greater than 20 minutes for ammonia and cyclohexane, and greater than 15 minutes for sulfur dioxide.

It will be understood that the layer thicknesses 12, 22, 32 can be selected, tailored, or the like for a desired filter performance with respect to selected agents. In one non-limiting example, reducing the first layer thickness 12 can decrease filter performance with respect to nitrogen dioxide and ammonia, while providing for overall weight reduction of the sorbent filter 5. In another example, increasing any of the first, second, or third layer thicknesses 12, 22, 32 can increase filter performance while also increasing flow resistance through the sorbent filter 5.

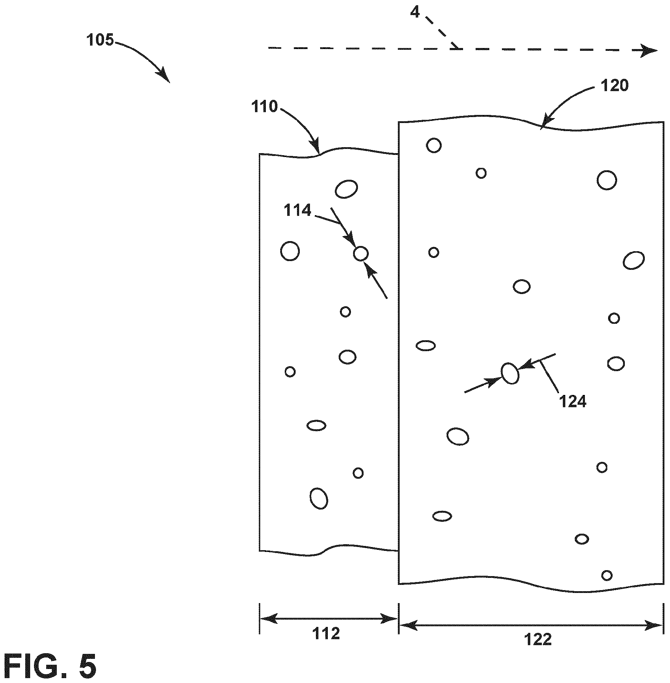

Referring now to FIG. 5, another sorbent filter 105 is illustrated that is suitable for use in the air filter assembly 1 (FIG. 1). The sorbent filter 105 is similar to the sorbent filter 5; therefore, like parts will be described with like numerals increased by 100, with it being understood that the description of the like parts of the sorbent filter 5 applies to the sorbent filter 105, except where noted.

The sorbent filter 105 is illustrated having a first adsorbent layer 110 and a second adsorbent layer 120 (also referred to as "first layer 110" and "second layer 120"). One difference compared to the sorbent filter 5 is that the sorbent filter 105 does not include a third adsorbent layer. The air flow path 4 is illustrated in a direction from the first layer 110 toward the second layer 120.

The first layer 110 and the second layer 120 can define a respective first layer thickness 112 and second layer thickness 122. It will be understood that the layer thicknesses 112, 122, can have any suitable size, including any suitable relative size. In some non-limiting examples: the first layer thickness 112 can be between 2-6 mm, including 2.3 mm; and the second layer thickness 122 can be between 8-10 mm, including 10 mm. In addition, an exemplary first particle size 114 and second particle size 124 are shown for the respective first layer 110 and second layer 120. In a non-limiting example, either or both of the first particle size 114 or the second particle size 124 can be in a range between 0.212-2.36 mm.

The first layer 110 can include an impregnated metal organic framework (MOF). More specifically, it is contemplated that the first layer 110 can include a zirconium-based MOF impregnated with zinc chloride in an amount by weight greater than 0%, and less than or equal to 10%. The zirconium-based MOF can be additionally impregnated with water, in an amount by weight greater than 0%, and less than or equal to 20%. The zirconium-based MOF can be additionally impregnated with a polymeric binder, in an amount by weight greater than 0%, and less than or equal to 10%.

The second layer 120 is positioned adjacent the first layer 110 and includes an adsorbent material. More specifically, the adsorbent material can include activated carbon, in an amount by weight greater than or equal to 60%, and less than 100%. The adsorbent material can also include copper II oxide, in an amount by weight greater than 0%, and less than or equal to 10%. The adsorbent material can also include silver, in an amount by weight greater than 0%, and less than or equal to 5%. The adsorbent material can also include triethylenediamine, in an amount by weight greater than or equal to 3.5%, and less than or equal to 15%. The adsorbent material can further include water, in an amount by weight greater than or equal to 0%, and less than or equal to 10%.

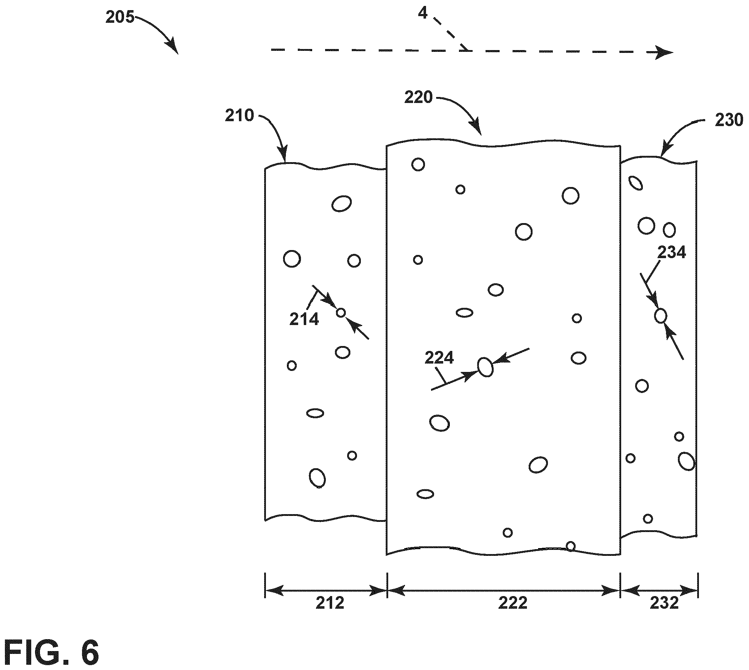

Referring now to FIG. 6, another sorbent filter 205 is illustrated that is suitable for use in the air filter assembly 1 (FIG. 1). The sorbent filter 205 is similar to the sorbent filters 5, 105; therefore, like parts will be described with like numerals further increased by 100, with it being understood that the description of the like parts of the sorbent filter 5, 105 applies to the sorbent filter 205, except where noted.

The sorbent filter 205 is illustrated having a first adsorbent layer 210, a second adsorbent layer 220, and a third adsorbent layer 230 (also referred to as "first layer 210," "second layer 220," and "third layer 230"). The air flow path 4 is illustrated in a direction from the first layer 210 toward the second layer 220.

The first layer 210, second layer 220, and third layer 230 can define a respective first layer thickness 212, second layer thickness 222, and third layer thickness 232. It will be understood that the layer thicknesses 212, 222, 232 can have any suitable size, including any suitable relative size. In some non-limiting examples: the first layer thickness 212 can be between 2-6 mm, including 3 mm; the second layer thickness 222 can be between 14-21 mm, including 21 mm; and the third layer thickness 232 can be between 2-10 mm, including 3 mm. In addition, an exemplary first particle size 214, second particle size 224, and third particle size 234 are shown for the respective first layer 210, second layer 220, and third layer 230. In a non-limiting example, any or all of the first particle size 214, second particle size 224, or third particle size 234 can be in a range between 0.212-2.36 mm.

The first layer 210 can include an impregnated metal organic framework (MOF). More specifically, it is contemplated that the first layer 210 can include a UiO-66 NH₂-based MOF impregnated with magnesium chloride in an amount by weight greater than 0%, and less than or equal to 10%. The UiO-66 NH₂-based MOF can be additionally impregnated with a polymeric binder, in an amount by weight greater than 0%, and less than or equal to 10%.

The second layer 220 is positioned adjacent the first layer 210 and includes an adsorbent material. More specifically, the adsorbent material can include activated carbon, in an amount by weight greater than or equal to 54%, and less than 100%. The adsorbent material can also include copper II oxide, in an amount by weight greater than 0%, and less than or equal to 10%. The adsorbent material can also include zinc oxide, in an amount by weight greater than 0%, and less than or equal to 10%. The adsorbent material can also include silver, in an amount by weight greater than 0%, and less than or equal to 1%. The adsorbent material can also include triethylenediamine, in an amount by weight greater than 0%, and less than or equal to 15%. The adsorbent material can further include water, in an amount by weight greater than or equal to 0%, and less than or equal to 10%.

The third layer 230 is positioned adjacent the second layer 220 and also includes an adsorbent material. More specifically, the third layer 230 can include activated carbon, in an amount by weight greater than or equal to 80%, and less than 100%. The third layer 30 can also include copper carbonate hydroxide, in an amount by weight greater than 0%, and less than or equal to 10%. The third layer 30 can further include copper oxide, in an amount by weight greater than 0%, and less than or equal to 10%.

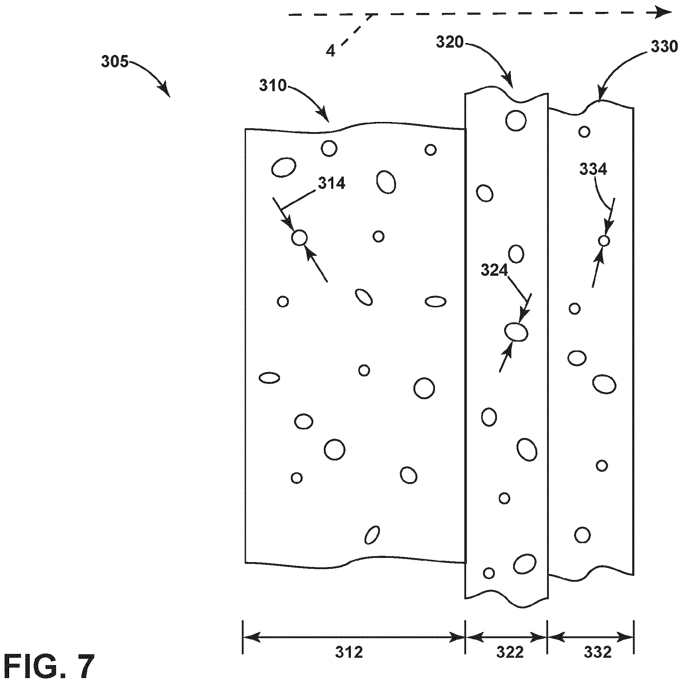

Referring now to FIG. 7, another sorbent filter 305 is illustrated that is suitable for use in the air filter assembly 1 (FIG. 1). The sorbent filter 305 is similar to the sorbent filters 5, 105, 205; therefore, like parts will be described with like numerals further increased by 100, with it being understood that the description of the like parts of the sorbent filter 5, 105, 205 applies to the sorbent filter 305, except where noted.

The sorbent filter 305 is illustrated having a first adsorbent layer 310, a second adsorbent layer 320, and a third adsorbent layer 330 (also referred to as "first layer 310," "second layer 320," and "third layer 330"). The air flow path 4 is illustrated in a direction from the first layer 310 toward the second layer 320.

The first layer 310, second layer 320, and third layer 330 can define a respective first layer thickness 312, second layer thickness 322, and third layer thickness 332. It will be understood that the layer thicknesses 312, 322, 332 can have any suitable size, including any suitable relative size. In some non-limiting examples: the first layer thickness 312 can be 14 mm, the second layer thickness 322 can be 4 mm; and the third layer thickness 332 can be 7 mm. In addition, an exemplary first particle size 314, second particle size 324, and third particle size 334 are shown for the respective first layer 310, second layer 320, and third layer 330. In a non-limiting example, any or all of the first particle size 314, second particle size 324, or third particle size 334 can be in a range between 0.212-2.36 mm.

The first layer 310 can include impregnated activated carbon, such as Chemsorb MilSpec from Molecular Products, Inc. in one non-limiting example. It is contemplated that the first layer 310 can include activated carbon impregnated with copper II oxide, in an amount by weight greater than 0%, and less than or equal to 10%. The first layer 310 can additionally include silver, in an amount by weight greater than 0%, and less than or equal to 1%. The first layer 310 can additionally include triethylenediamine, in an amount by weight greater than 0%, and less than or equal to 10%. The first layer 310 can additionally include zinc oxide, in an amount by weight greater than 0%, and less than or equal to 10%.

The second layer 320 is positioned adjacent the first layer 310 and includes an adsorbent material. More specifically, the adsorbent material can include activated carbon, in an amount by weight greater than or equal to 55%, and less than 100%. The adsorbent material can also include copper carbonate, in an amount by weight greater than 0%, and less than or equal to 10%. The adsorbent material can also include copper oxide, in an amount by weight greater than 0%, and less than or equal to 10%. The adsorbent material can also include triethylenediamine, in an amount by weight greater than or equal to 0%, and less than or equal to 15%. The adsorbent material can further include water, in an amount by weight greater than or equal to 0%, and less than or equal to 10%.

The third layer 330 is positioned adjacent the second layer 320 and also includes an adsorbent material. The third layer 330 can include activated carbon, such as Chemsorb 620 from Molecular Products, Inc. in one non-limiting example. More specifically, the third layer 330 can be impregnated with zinc chloride in an amount greater than 0%, and less than or equal to 25%.

The foregoing results altogether document the excellent protective performance of the sorbent filter described herein. The use of water or moisture content in one or more layers reduces dust accumulation and improves breathability, and also extends a working lifetime of the sorbent filter compared to traditional filters. The use of impregnated MOFs provides for significant improvements in adsorption characteristics compared to traditional filters, with resulting filter performance being significantly improved over the prior art.

To the extent not already described, the different features and structures of the various embodiments can be used in combination, or in substitution with each other as desired. That one feature is not illustrated in all of the embodiments is not meant to be construed that it cannot be so illustrated, but is done for brevity of description. Thus, the various features of the different embodiments can be mixed and matched as desired to form new embodiments, whether the new embodiments are expressly described. All combinations or permutations of features described herein are covered by this disclosure.

This written description uses examples to disclose the present disclosure, including the best mode, and also to enable any person skilled in the art to practice the disclosure, including making and using any devices or systems and performing any incorporated methods. The patentable scope of the disclosure is defined by the claims, and may include other examples that occur to those skilled in the art. Such other examples are intended to be within the scope of the claims if they include structural elements that do not differ from the literal language of the claims, or if they include equivalent structural elements with insubstantial differences from the literal languages of the claims.

Further aspects of the disclosure are provided in the following clauses:

A sorbent filter, comprising: a first adsorbent layer comprising at least one of a metal organic framework (MOF) or activated carbon; and a second adsorbent layer positioned adjacent the first adsorbent layer and comprising an adsorbent material with water in an amount by weight greater than 0%, and less than or equal to 10%.

A sorbent filter, comprising: a first adsorbent layer comprising a metal organic framework with water in an amount by weight greater than 0%, and less than or equal to 20%; and a second adsorbent layer positioned adjacent the first adsorbent layer and comprising activated carbon with water in an amount by weight greater than or equal to 0%, and less than or equal to 10%.

The sorbent filter of any preceding clause, wherein the first adsorbent layer comprises the MOF, and wherein the MOF is one of zirconium-based or UiO-66 NH₂-based.

The sorbent filter of any preceding clause, wherein the MOF further comprises at least one of zinc chloride, magnesium chloride, a polymeric binder, or water to define an impregnated MOF.

The sorbent filter of any preceding clause, wherein the impregnated MOF comprises zinc chloride.

The sorbent filter of any preceding clause, wherein the impregnated MOF comprises water in an amount by weight greater than 0%, and less than or equal to 20%.

The sorbent filter of any preceding clause, wherein the impregnated MOF comprises: zinc chloride in an amount by weight greater than 0% and less than or equal to 10%; water in an amount by weight greater than 0%, and less than or equal to 20%; and polymeric binder in an amount by weight greater than 0%, and less than or equal to 10%.

The sorbent filter of any preceding clause, wherein the impregnated MOF comprises: UiO-66 NH₂ in an amount by weight greater than or equal to 80%, and less than or equal to 100%; and polymeric binder in an amount by weight greater than 0%, and less than or equal to 10%.

The sorbent filter of any preceding clause, wherein the first adsorbent layer comprises: the activated carbon; copper II oxide in an amount by weight greater than 0%, and less than or equal to 10%; silver in an amount by weight greater than 0%, and less than or equal to 1%; triethylenediamine in an amount by weight greater than 0%, and less than or equal to 10%; and zinc oxide in an amount by weight greater than 0%, and less than or equal to 10%.

The sorbent filter of any preceding clause, wherein the adsorbent material of the second adsorbent layer comprises activated carbon, copper II oxide, triethylenediamine, water, and at least one of molybdenum, silver, or zinc oxide.

The sorbent filter of any preceding clause, wherein the adsorbent material of the second adsorbent layer comprises: activated carbon in an amount by weight greater than or equal to 60%, and less than 100%; copper II oxide in an amount by weight greater than 0%, and less than or equal to 10%; molybdenum in an amount by weight greater than 0%, and less than or equal to 5%; triethylenediamine in an amount by weight greater than or equal to 0%, and less than or equal to 15%; and water in an amount by weight greater than or equal to 0%, and less than or equal to 10%.

The sorbent filter of any preceding clause, wherein the adsorbent material of the second adsorbent layer comprises: activated carbon in an amount by weight greater than or equal to 60%, and less than 100%; copper II oxide in an amount by weight greater than 0%, and less than or equal to 10%; silver in an amount by weight greater than 0%, and less than or equal to 5%; triethylenediamine in an amount by weight greater than or equal to 0%, and less than or equal to 15%; and water in an amount by weight greater than or equal to 0%, and less than or equal to 10%.

The sorbent filter of claim 9, wherein the adsorbent material of the second adsorbent layer comprises: activated carbon in an amount by weight greater than or equal to 54%, and less than 100%; copper II oxide in an amount by weight greater than 0%, and less than or equal to 10%; silver in an amount by weight greater than 0%, and less than or equal to 1%; zinc oxide in an amount by weight greater than 0%, and less than or equal to 10%; triethylenediamine in an amount by weight greater than 0%, and less than or equal to 15%; and water in an amount by weight greater than or equal to 0%, and less than or equal to 10%.

The sorbent filter of any preceding clause, further comprising a third adsorbent layer comprising at least one of activated carbon, copper carbonate hydroxide, zinc oxide, water, or copper oxide.

The sorbent filter of any preceding clause, wherein the third adsorbent layer comprises: activated carbon in an amount by weight greater than or equal to 60%, and less than 100%; copper carbonate hydroxide in an amount by weight greater than 0%, and less than or equal to 10%; copper oxide in an amount by weight greater than 0%, and less than or equal to 10%; water in an amount by weight greater than 0%, and less than or equal to 10%; and zinc oxide in an amount by weight greater than 0%, and less than or equal to 10%.

The sorbent filter of any preceding clause, wherein the first adsorbent layer defines a first layer thickness, and the second adsorbent layer defines a second layer thickness greater than the first layer thickness.

The sorbent filter of any preceding clause, further comprising a third adsorbent layer positioned adjacent the second adsorbent layer and comprising at least one of activated carbon, copper carbonate hydroxide, or copper oxide, and defining a third layer thickness less than the second layer thickness.

The sorbent filter of any preceding clause, wherein the MOF is one of zirconium-based or UiO-66 NH₂-based, and further comprises at least one of zinc chloride, magnesium chloride, or a polymeric binder.

The sorbent filter of any preceding clause, wherein the second adsorbent layer comprises activated carbon, copper II oxide, triethylenediamine, water, and at least one of molybdenum, silver, or zinc oxide.

An air filter assembly, comprising: a housing at least partially defining a filtering facepiece respirator with an air flow path; and the sorbent filter of any preceding clause disposed within the housing; wherein the air flow path is in a direction from one of the first adsorbent layer or the second adsorbent layer toward the other of the first adsorbent layer or the second adsorbent layer.

The air filter assembly of any preceding clause, wherein the air flow path is in a direction from the first adsorbent layer toward the second adsorbent layer.

The air filter assembly of any preceding clause, wherein the air flow path is in a direction from the second adsorbent layer toward the first adsorbent layer.

## Claims

1. A sorbent filter (5, 105, 205, 305), comprising:
a first adsorbent layer (10, 110, 210, 310) comprising at least one of a metal organic framework (MOF) or activated carbon; and
a second adsorbent layer (20, 120, 220, 320) positioned adjacent the first adsorbent layer (10, 110, 210, 310) and comprising an adsorbent material with water in an amount by weight greater than 0%, and less than or equal to 10%.

2. The sorbent filter (5, 105, 205, 305) of claim 1, wherein the first adsorbent layer (10, 110, 210, 310) comprises the MOF, and wherein the MOF is one of zirconium-based or UiO-66 NH2-based.

3. The sorbent filter (5, 105, 205, 305) of claim 2, wherein the MOF further comprises at least one of zinc chloride, magnesium chloride, a polymeric binder, or water to define an impregnated MOF.

4. The sorbent filter (5, 105, 205, 305) of claim 3, wherein the impregnated MOF comprises zinc chloride.

5. The sorbent filter (5, 105, 205, 305) of any of claims 1-4, wherein the impregnated MOF comprises water in an amount by weight greater than 0%, and less than or equal to 20%.

6. The sorbent filter (5, 105, 205, 305) of any of claims 1-5, wherein the impregnated MOF comprises:
zinc chloride in an amount by weight greater than 0% and less than or equal to 10%;
water in an amount by weight greater than 0%, and less than or equal to 20%; and
polymeric binder in an amount by weight greater than 0%, and less than or equal to 10%.

7. The sorbent filter (5, 105, 205, 305) of any of claims 1-5, wherein the impregnated MOF comprises:
UiO-66 NH2 in an amount by weight greater than or equal to 80%, and less than or equal to 100%; and
polymeric binder in an amount by weight greater than 0%, and less than or equal to 10%.

8. The sorbent filter (5, 105, 205, 305) of any of claims 1-7, wherein the first adsorbent layer (10, 110, 210, 310) comprises:
the activated carbon;
copper II oxide in an amount by weight greater than 0%, and less than or equal to 10%;
silver in an amount by weight greater than 0%, and less than or equal to 1%;
triethylenediamine in an amount by weight greater than 0%, and less than or equal to 10%; and
zinc oxide in an amount by weight greater than 0%, and less than or equal to 10%.

9. The sorbent filter (5, 105, 205, 305) of any of claims 1-8, wherein the adsorbent material of the second adsorbent layer (20, 120, 220, 320) comprises activated carbon, copper II oxide, triethylenediamine, water, and at least one of molybdenum, silver, or zinc oxide.

10. The sorbent filter (5, 105, 205, 305) of claim 9, wherein the adsorbent material of the second adsorbent layer (20, 120, 220, 320) comprises:
activated carbon in an amount by weight greater than or equal to 60%, and less than 100%;
copper II oxide in an amount by weight greater than 0%, and less than or equal to 10%;
molybdenum in an amount by weight greater than 0%, and less than or equal to 5%;
triethylenediamine in an amount by weight greater than or equal to 0%, and less than or equal to 15%; and
water in an amount by weight greater than or equal to 0%, and less than or equal to 10%.

11. The sorbent filter (5, 105, 205, 305) of claim 9, wherein the adsorbent material of the second adsorbent layer (20, 120, 220, 320) comprises:
activated carbon in an amount by weight greater than or equal to 60%, and less than 100%;
copper II oxide in an amount by weight greater than 0%, and less than or equal to 10%;
silver in an amount by weight greater than 0%, and less than or equal to 5%;
triethylenediamine in an amount by weight greater than or equal to 0%, and less than or equal to 15%; and
water in an amount by weight greater than or equal to 0%, and less than or equal to 10%.

12. The sorbent filter (5, 105, 205, 305) of claim 9, wherein the adsorbent material of the second adsorbent layer (20, 120, 220, 320) comprises:
activated carbon in an amount by weight greater than or equal to 54%, and less than 100%;
copper II oxide in an amount by weight greater than 0%, and less than or equal to 10%;
silver in an amount by weight greater than 0%, and less than or equal to 1%;
zinc oxide in an amount by weight greater than 0%, and less than or equal to 10%;
triethylenediamine in an amount by weight greater than 0%, and less than or equal to 15%; and
water in an amount by weight greater than or equal to 0%, and less than or equal to 10%.

13. The sorbent filter (5, 105, 205, 305) of any of claims 1-12, further comprising a third adsorbent layer (30, 230, 330) comprising at least one of activated carbon, copper carbonate hydroxide, zinc oxide, water, or copper oxide.

14. The sorbent filter (5, 105, 205, 305) of claim 13, wherein the third adsorbent layer (30, 230, 330) comprises:
activated carbon in an amount by weight greater than or equal to 60%, and less than 100%;
copper carbonate hydroxide in an amount by weight greater than 0%, and less than or equal to 10%;
copper oxide in an amount by weight greater than 0%, and less than or equal to 10%;
water in an amount by weight greater than 0%, and less than or equal to 10%; and
zinc oxide in an amount by weight greater than 0%, and less than or equal to 10%.

15. An air filter assembly (1), comprising:
a housing (2) at least partially defining a filtering facepiece respirator (6) with an air flow path (4); and
the sorbent filter (10, 110, 210, 310) of any of claims 1-14 disposed within the housing (2);
wherein the air flow path (4) is in a direction from one of the first adsorbent layer (10, 110, 210, 310) or the second adsorbent layer (20, 120, 220, 320) toward the other of the first adsorbent layer (10, 110, 210, 310) or the second adsorbent layer (20, 120, 220, 320).
